# EUROPÄISCHE PATENTSCHRIFT

(11) **EP 1 962 922 B1**
(45) Veröffentlichungstag und Bekanntmachung des Hinweises auf die Patenterteilung: **14.10.2009**
(21) Anmeldenummer: 06817768.2
(22) Anmeldetag: 21.12.2006
(51) Int. Cl.: A61M 1/06

(54) **Brusthaube mit Widerstandsheizelement aus Kunststoff**
breast cup with resistive heating element made out of plastic
bonnet pour sein avec élément résistif en plastique

(30) Priorität: 22.12.2005 CH 20452005
(43) Veröffentlichungstag der Anmeldung: 03.09.2008
(73) Patentinhaber: Medela Holding AG, 6340 Baar (CH)
(72) Erfinder: THOMMEN, Daniel, CH-6312 Steinhausen (CH); FURRER, Simon, CH-6005 Luzern (CH); LARSSON, Michael, CH-6300 Zug (CH)
(74) Vertreter: Clerc, Natalia
(86) Internationale Anmeldenummer: PCT/CH2006/000718
(87) Internationale Veröffentlichungsnummer: WO 2007/071093

(56) Entgegenhaltungen:
- WO-A-00/47247
- WO-A2-01/95841
- WO-A2-20/05070476
- US-A1- 2003 073 951
- US-A1- 2003 121 899
- US-A1- 2003 191 432
- US-A1- 2004 087 898

## Beschreibung

### Technisches Gebiet

Die Erfindung betrifft eine Brusthaube gemäss Oberbegriff des Patentanspruchs 1.

### Stand der Technik

Brustpumpen zum Abpumpen menschlicher Muttermilch sind bekannt. Sie weisen üblicherweise eine Vakuumpumpe, eine oder zwei damit verbindbare Brusthauben zur Auflage auf eine Mutterbrust bzw. beide Mutterbrüste sowie je einen mit der Brusthaube verbindbaren Milchsammelbehälter zum Sammeln der abgepumpten Muttermilch auf.

Es ist ferner bekannt, dass das Aufbringen von Wärme auf die Brust vor und während dem Pumpvorgang nicht nur den Komfort für die Mutter erhöht, sondern auch die Milchgänge erweitert, Stauungen zu lösen hilft und die Areola weicher macht.

So offenbart US 5'897'580 einen c-förmigen Brusthaubeneinsatz mit einer chemischen Füllung, welche eine exothermische Reaktion durchlaufen kann. WO 2005/070476 offenbart einen elektrisch beheizbaren Brusthaubeneinsatz, wobei die elektrischen Widerstandsheizdrähte entweder im Brusthaubeneinsatz selber oder im Brusthaubentrichter angeordnet sind.

US 2002/0198489 offenbart eine Brusthaube mit einem doppelwandigen Brusthaubentrichter. Durch den dadurch entstandenen Zwischenraum kann ein Fluid, beispielsweise warmes Wasser, geleitet werden, um die Brusthaube zu wärmen. US 6'358'226 schlägt vor, warme Luft in eine doppelwandige Brusthaube zu pumpen.

US 2004/0087898 beschreibt eine Brusthaube mit einem elektrischen Widerstandsheizdraht, welcher entweder auf der äusseren oder inneren Oberfläche der Brusthaube aufgebracht ist oder in ihrem Material eingebettet ist.

WO 01/95841 A2 offenbart eine heizbare Unterlage für Patienten, bei der das Widerstandheizelement aus Kunststoff gefertigt ist.

Heizbare Brusthaubeneinsätze weisen den Nachteil auf, dass die Mutter ein weiteres separates Teil am Brusthaubenset montieren muss, bevor sie mit dem Abpumpen beginnen kann. Dies ist unter Zeitdruck oder nachts nicht wünschenswert. Brusthauben mit elektrischen Widerstandsheizdrähten sind aufwendig in der Herstellung. Es ist insbesondere schwierig, Drahtgeflechte einzulegen und zu umspritzen. Des weiteren besteht die Gefahr, dass sich die Drähte bei unsachgemässen Gebrauch von der Brusthaube lösen können. Auch kann ihr Anblick die Mutter verunsichern, da sie sich bewusst wird, dass sie elektrischen Strom an die Brust führt. Des weiteren versperren die Heizwicklungen der Mutter die Sicht auf die Brustwarze und den umliegenden Bereich, so dass sie den Abpumpvorgang schlecht beobachten kann.

### Darstellung der Erfindung

Es ist deshalb eine Aufgabe der Erfindung, eine alternative Vorrichtung zur Erwärmung der Mutterbrust zu schaffen, welche vor und während dem Abpumpen verwendet werden kann.

Diese Aufgabe löst eine Brusthaube mit den Merkmalen des Patentanspruchs 1.

Die erfindungsgemässe Brusthaube zur Verwendung mit einer Brustpumpe zum Abpumpen von Muttermilch weist einen Brusthaubentrichter zur Anlage an eine Mutterbrust, einen ersten Kopplungsteil zur Verbindung mit der Brustpumpe und einen zweiten Kopplungsteil zur Verbindung mit einem Milchsammelbehälter auf. Die Brusthaube weist ferner ein elektrisches Widerstandsheizelement zu ihrer Erwärmung auf, wobei das elektrische Widerstandsheizelement aus einem elektrisch leitenden Kunststoffmaterial gefertigt ist. Die Brusthaube ist in einem Zwei- oder Mehrkomponentenspritzgussverfahren hergestellt.

In einer bevorzugten Ausführungsform ist das elektrisch leitende Kunststoffmaterial ein Kunststoffbasismaterial und enthält einen elektrisch leitenden Zusatzstoff.

Die erfindungsgemässe Brusthaube weist den Vorteil auf, dass sie keinen Einsatz benötigt, welcher beheizt werden muss. Zudem kann der Brusthaubentrichter weiterhin relativ schmal ausgeführt werden, da keine Kammer benötigt wird, um ein Heizmedium aufzunehmen. Dank dem Zwei- oder Mehrkomponentenspritzgussverfahren ist das Widerstandheizelement praktisch unlösbar mit der restlichen Brusthaube verbunden, so dass es auch bei grösserer Beanspruchung keinen Schaden nimmt. Ein weiterer Vorteil ist, dass sich das Widerstandsheizelement einfacher auf bzw. in das Material der Brusthaube einarbeiten lässt, so dass seine Platzierung auf der Brusthaube und auch seine Formgestaltung flexibler sind und mehr Wahlmöglichkeiten offen lassen. Des weiteren kann das Widerstandsheizelement so gestaltet werden, dass es nicht auf den ersten Blick als stromführend identifiziert wird.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine perspektivische Darstellung eines Brustpumpensets mit Brustpumpe und Brusthaube gemäss der Erfindung in einer ersten Ausführungsform;
- Figur 2a: eine perspektivische Darstellung einer erfindungsgemässen Brusthaube in einer zweiten Ausführungsform;
- Figur 2b: eine andere perspektivische Darstellung der Brusthaube gemäss Figur 2a und
- Figur 3: eine perspektivische Darstellung eines zweiteiligen Brusthaubentrichters gemäss einer dritten Ausführungsform der Erfindung.

### Wege zur Ausführung der Erfindung

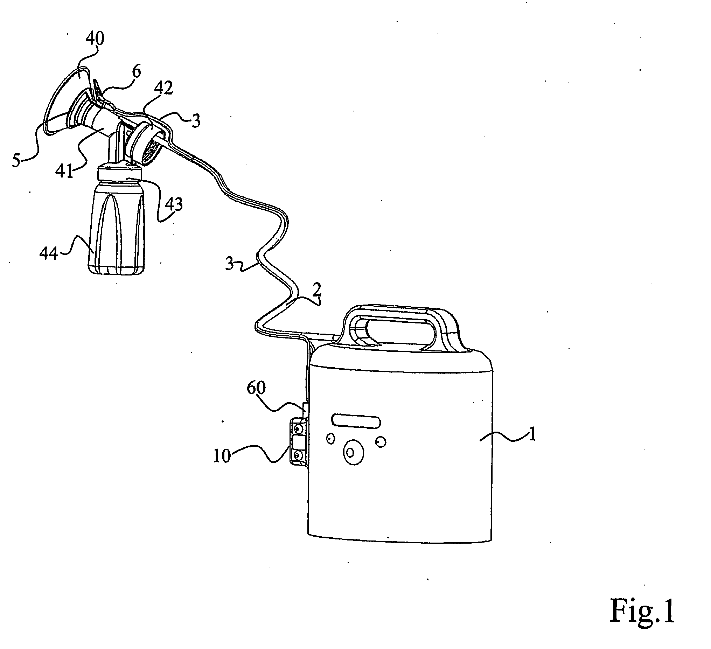

In Figur 1 ist ein Brustpumpenset im für seine Verwendung zusammengesetzten Zustand dargestellt. Das Brustpumpenset umfasst eine Brustpumpe 1 in Form einer in einem Gehäuse angeordneten motorbetriebenen Vakuumpumpe oder Saugpumpe, eine oder zwei Brusthauben 4, pro Brusthaube je einen mit dieser verbindbaren Milchsammelbehälter 44 und pro Brusthaube 4 je eine Saugleitung 2 zur Verbindung der Brustpumpe 1 mit der entsprechenden Brusthaube 4.

Das dargestellte Brustpumpenset ist lediglich ein Ausführungsbeispiel einer Vielzahl von Möglichkeiten. Anstelle der motorbetriebenen Vakuumpumpe kann sie auch eine Handpumpe umfassen, welche über eine Saugleitung oder direkt an der Brusthaube angeschlossen ist. Auch die Form der Brusthaube kann variieren. Als Milchsammelbehälter eignen sich alle geeigneten Gefässe oder Beutel.

Die Brusthaube 4 weist einen Brusthaubentrichter 40 auf, welcher auf die Brust aufgelegt wird, so dass sie vorzugsweise die Brustwarze umschliesst, diese aber freilässt. Anschliessend an den Brusthaubentrichter 40 folgt ein Brusthaubenhals 41, welcher in einem ersten Kopplungsteil 42 endet. In einem Winkel dazu zweigt ein zweiter Kopplungsteil 43 vom Brusthaubenhals 41 ab. Der erste Kopplungsteil 42 dient zum Anschluss der Saugleitung 2. Im hier dargestellten Beispiel kann alternativ eine Handpumpe oder eine kleine motorbetriebene, insbesondere eine batteriebetriebe Vakuumpumpe angeschlossen, beispielsweise angeschraubt werden. Am zweiten Kopplungsteil 43 ist der Milchsammelbehälter 44 in Form einer Flasche angeschraubt. Alternativ kann auch ein Milchsammelbeutel befestigt werden. Im hier dargestellten Beispiel ist die Brusthaube einstückig gefertigt und besteht vorzugsweise aus einem starren Kunststoffmaterial, insbesondere aus Polypropylen (PP).

Die Brusthaube 4 ist mit einem elektrischen Widerstandsheizelement 5 versehen. Vorzugsweise ist dieses Widerstandsheizelement 5 im Bereich des Brusthaubentrichters 40 angeordnet. Es kann jedoch auch am Hals oder je nach Form der Brusthaube 4 an einer anderen Stelle angeordnet sein, solange mit ihm genügend Wärme in den Bereich des Brusthaubentrichters 40 gebracht werden kann. Das Widerstandsheizelement 5 verläuft vorzugsweise spiralförmig oder, wie hier dargestellt, in konzentrischen Kreisen auf dem Brusthaubentrichter 40. Andere Verlegearten des Widerstandheizelements sind jedoch auch möglich. Es kann, wie hier dargestellt ist, auf der äusseren Oberfläche der Brusthaube 4 angeordnet sein. Ebenso kann es jedoch auf der inneren Oberfläche verlaufen oder im Material der Brusthaube 4 eingebettet sein. Zur Aktivierung des Widerstandsheizelements 4 ist eine elektrische Leitung 3 zur Brustpumpe 1 vorhanden. Vorzugsweise ist diese elektrische Leitung 3 mit der Saugleitung 2 verbunden und sie teilt sich nur in den Endbereichen der Leitung. Das heisst die elektrische Leitung 3 weist einen eigenen elektrischen Anschluss bzw. ein eigenes erstes Kontaktelement 6 im Bereich des Brusthaubentrichters 40 und ein eigenes zweites Kontaktelement 60 an das Gehäuse der Brustpumpe 1 auf. Vorzugsweise sind es Steckverbindungen. Im hier dargestellten Beispiel ist das elektrische Modul zur Bedienung der Heizung bzw. des Widerstandheizelements 5 ein am Hauptgehäuse der Brustpumpe 1 extern angebrachtes Modul 10. Selbstverständlich kann es jedoch in das Hauptgehäuse der Brustpumpe integriert und die entsprechenden Bedienungsknöpfe, -tasten oder -felder am Hauptgehäuse angeordnet sein.

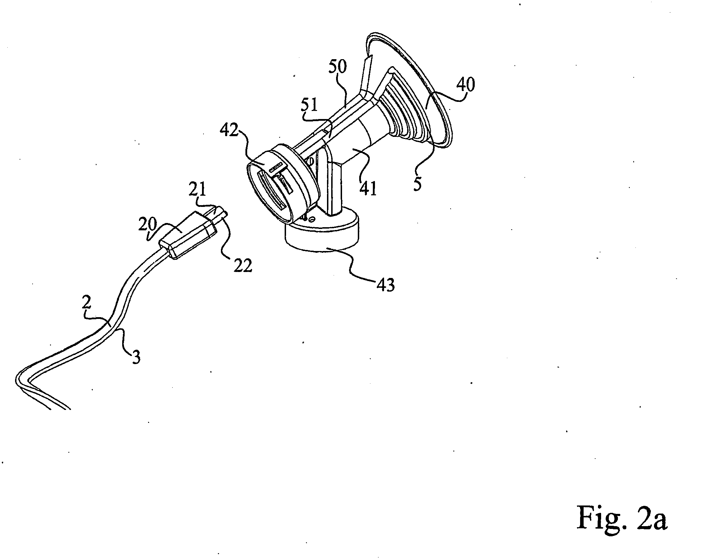

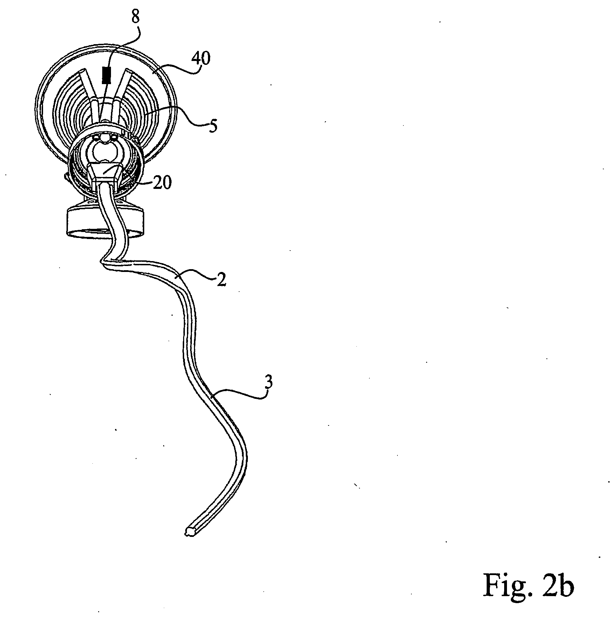

In den Figuren 2a und 2b ist eine weitere Ausführungsform dargestellt. Hier ist ein gemeinsamer Stecker 20 für die Saugleitung 2 und die elektrische Leitung 3 vorhanden, so dass im ersten Kopplungsteil 42 der elektrische Anschluss 22 benachbart zum Anschluss 21 der Saugleitung 2 angeordnet ist. Entsprechend sind auf der Brusthaube 4 Anschlussleitungen 50, 51 vorhanden, welche den elektrischen Anschluss 22 mit dem Widerstandsheizelement 5 verbinden.

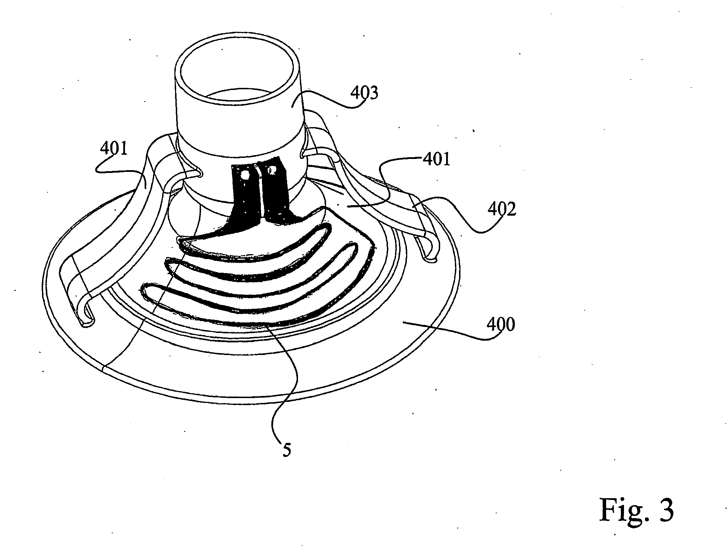

In Figur 3 ist eine weitere Ausführungsform dargestellt, wobei nur der Brusthaubentrichter 40 gezeigt ist. Hier ist die Brusthaube 4 nicht wie in den vorhegehenden Beispielen einstückig gefertigt sondern mehrteilig. Der Brusthaubentrichter 40 ist als Einsteckelement ausgebildet, welches in den Brusthaubenhals eingesteckt werden kann. Hierzu verfügt der Brusthaubentrichter 40 selber über einen kurzen Hals 403. Der Brusthaubentrichter 40 weist einen starren vorderen, d.h. der Mutter am nächsten liegenden Bereich 400, einen weichen mittleren Bereich 401 und den starren Hals 403 auf. Hals 403 und vorderer Bereich 400 sind über starre Stege 402 miteinander fixiert. Derartige Brusthauben sind bekannt. Ihr Vorteil ist, dass sich der mittlere Bereich optimal an die Mutterbrust anschmiegt Üblicherweise besteht der harte Bereich 400 aus Polypropylen (PP) und der weiche Bereich 401 aus einem thermoplastischen Elastomer (TPE), beispielsweise aus Thermolast K. Neu ist nun, dass im mittleren, weichen Bereich 401 das Widerstandsheizelement 5 angeordnet ist. Selbstverständlich können auch der Brusthaubenhals 41 und die Kopplungsteile 42, 43 einstückig mit dem starren Hals 403 des Brusthaubentrichters 40 verbunden sein.

Das elektrische Widerstandsheizelement 5 zur Erwärmung der Brust ist nun erfindungsgemäss in allen oben genannten Beispielen aus einem elektrisch leitenden Kunststoffmaterial gefertigt, das heisst aus einem Kunststoff, welcher entweder selber elektrisch leitend ist oder mittels Zusatzstoffen elektrisch leitend gemacht wurde.

Als selber elektrisch leitende Kunststoffe eignet sich Baytron P.

Wurde der Kunststoff elektrisch leitend gemacht, so weist er ein Kunststoftbasismaterial und einen elektrisch leitenden Zusatzstoff auf. Als Kunststoffbasismaterial wird vorzugsweise, aber nicht ausschliesslich, Polyamid (PA), Polypropylen (PP) oder Polyethylen (PE) verwendet.

Der Zusatzstoff ist vorzugsweise faserformig. Werden Fasern verwendet, so weisen sie vorzugsweise eine Länge von 100 bis 300 µm auf. Als Fasern eignen sich insbesondere Kohlefasern, Kupferfasern und Eisenfasern. Der Zusatzstoff kann aus einer einzigen Sorte dieser Fasern bestehen oder aus einem Gemisch davon.

Gute Resultate wurden mit Kunststoffen der Firma Albis (ALCOM PA66 910/1 CF10, ALCOM PA66 910/1 CF 30), mit Kunststoffen der Firma General Electric Plastics (PTC-B und PTC-B1) sowie mit Polypropylen der Firma Borealis, Typ BH 345 MO⁺ (mit 20 % Kohlenfasern) erzielt.

Der faserförmige Zusatzstoff ist vorzugsweise in einer Menge von 10% - 30% (Gewichtsprozent) dem Kunststoffbasismaterial beigemischt. Vorteil der faserförmigen Zusatzstoffe ist, dass sie bei der Erwärmung des Basismaterials die Bindung zueinander gehalten und somit ihre elektrische Leitfähigkeit nicht verlieren.

Der Zusatzstoff kann jedoch auch pulverförmig sein. Es eignen sich insbesondere Eisenpulver, Kupferpulver und Russ. Auch hier lassen sich die Pulverstoffe einzeln oder gemischt verwenden. Vorzugsweise weist das Pulver eine Korngrösse von 100 nm bis 1 µm auf.

Der pulverförmige Zusatzstoff ist vorzugsweise in einer Menge von 80% - 90% (Gewichtsprozent) dem Kunststoffbasismaterial beigemischt.

Vorzugsweise werden Materialien verwendet, welche einen spezifischen Widerstand von circa 2'500 Ωmm²/m aufweisen. Die erfindungsgemässe Brusthaube weist dann einen Widerstand von circa 20 Ω auf.

Die Brusthaube mit dem Widerstandsheizelement ist in einem Zwei oder Mehrkomponentenspritzgussverfahren hergestellt. Derartige Herstellungsverfahren sind bekannt und werden hier deshalb nicht im Detail beschrieben. Beim Zwei- oder Mehrkomponentenverfahren wird nach dem Erstarren der ersten Komponente im Spritzwerkzeug die Kavität vergrössert und eine zweite Komponente oder weitere Komponenten mit einem zweiten Spritzaggregat angespritzt. Die Vergrösserung kann entweder durch Schieber oder durch Wechsel der Kavität mittels eines Drehwerkzeugs oder eines Drehtisches erfolgen. Eine Komponente ist in diesem Fall das Grundmaterial der Brusthaube, eine zweite Komponente ist das Widerstandsheizelement. Je nach Bedarf können noch weitere Komponenten verwendet werden.

In einer bevorzugten Ausführungsform ist die Brusthaube ferner mit mindestens einem Sensor zur Temperaturüberwachung ausgestattet. Dieser Sensor kann bei Überhitzung einen Alarm auslösen bzw. ein Signal an eine Regelung des Heizelements liefern, um die Temperatur auf einen konstanten Wert bzw. einen konstanten Wertebereich zu regeln. Ein entsprechender Temperatursensor ist in Figur 2b mit der Bezugsnummer 8 versehen.

### Bezugszeichenliste

- 1.: Brustpumpe
- 10: Modul
- 2: Saugleitung
- 20: Stecker
- 21: Saugleitungsanschluss
- 22: Elektrischer Anschluss
- 3: Elektrische Leitung
- 4: Brusthaube
- 40: Brusthaubentrichter
- 41: Brusthaubenhals
- 42: Erster Kopplungsteil
- 43: Zweiter Kopplungsteil
- 44: Milchsammelbehälter
- 400: Starrer vorderer Bereich
- 401: Weicher mittlerer Bereich
- 402: Steg
- 403: Starrer Hals
- 5: Widerstandsheizelement
- 6: Erstes Kontaktelement
- 60: Zweites Kontaktelement
- 7: Stecker
- 8: Temperatursensor

## Patentansprüche

1. Brusthaube (4) zur Verwendung mit einer Brustpumpe (1) zum Abpumpen von Muttermilch, wobei die Brusthaube (4) einen Brusthaubentrichter (40) zur Anlage an eine Mutterbrust, einen ersten Kopplungsteil (42) zur Verbindung mit der Brustpumpe (1) und einen zweiten Kopplungsteil (43) zur Verbindung mit einem Milchsammelbehälter (44) aufweist und wobei die Brusthaube (4) ferner ein elektrisches Widerstandsheizelement (5) zur ihrer Erwärmung aufweist, **dadurch gekennzeichnet, dass** das elektrische Widerstandsheizelement (5) aus einem elektrisch leitenden Kunststoffmaterial gefertigt ist und wobei die Brusthaube (4) in einem Zwei- oder Mehrkomponentenspritzgussverfahren hergestellt ist.

2. Brusthaube nach Anspruch 1, wobei das elektrisch leitende Kunststoffmaterial ein Kunststoftbasismaterial und einen elektrisch leitenden Zusatzstoff enthält.

3. Brusthaube nach Anspruch 2, wobei der Zusatzstoff faserförming ist.

4. Brusthaube nach Anspruch 3, wobei die Fasern eine Länge von 100 bis 300 µm aufweisen.

5. Brusthaube nach einem der Ansprüche 2 bis 4, wobei der Zusatzstoff ein oder mehrere Materialien aus folgender Gruppe ist: Kohlefasern, Kupferfasern, Eisenfasern.

6. Brusthaube nach Anspruch 2, wobei der Zusatzstoff pulverförming ist.

7. Brusthaube nach Anspruch 6, wobei der Zusatzstoff ein oder mehrere Materialien aus folgender Gruppe ist: Eisenpulver, Kupferpulver, Russ.

8. Brusthaube nach einem der Ansprüche 2 bis 7, wobei das Kunststoffbasismaterial Polyamid (PA), Polypropylen (PP) oder Polyethylen (PE) ist.

9. Brusthauben nach einem der Ansprüche 2 bis 8, wobei der Zusatzstoff in einer Menge von 10% - 30% (Gewichtsprozent) dem Kunststoffbasismaterial beigemischt ist.

10. Brusthaube nach einem der Ansprüche 1 bis 9, wobei der Brosthaubentrichter (40) das Widerstandheizelement aufweist.

11. Brusthaube nach Anspruch 10, wobei das Widerstandsheizelement (5) im Material des Brusthaubentrichters (40) eingebettet ist.

12. Brusthaube nach Anspruch 10, wobei das Widerstandsheizelement (5) auf dem Material des Brusthaubentrichters (40) aufliegt.

13. Brosthaube nach Anspruch 10, wobei der Brusthaubentrichter (40) aus Polypropylen (PP), Polyamid (PA) oder Polyethylen (PE) gefertigt ist.

14. Brusthaube nach einem der Ansprüche 1 bis 13. wobei die Brusthaube (4) mit Ausnahme des Widerstandsheizelements (5) einstückig ausgebildet ist.

15. Brusthaube nach einem der Ansprüche 1 bis 13, wobei der Brusthaubentrichter (40) einen starren und einen weichen Bereich (400, 401) aufweist und wobei das Widerstandsheizelement (5) im weichen Bereich (401) angeordnet ist.

16. Brusthaube nach einem der Ansprüche 1 bis 15, wobei der Brosthaubentrichter (40) eine elektrische Steckverbindung (6) aufweist.

17. Brusthaube nach einem der Ansprüche 1 bis 15, wobei der erste Kopplungsteil (42) eine elektrische Steckverbindung (22) aufweist.

18. Brusthaube nach Anspruch 17, wobei die elektrische Steckverbindung (22) benachbart zu einem Sauganschluss (21) angeordnet ist.

19. Brusthaube nach einem der Ansprüche 1 bis 18, wobei das elektrische Widerstandsheizelement (5) spiral- oder kreisförmig auf dem Brusthaubentrichter (40) angeordnet ist.

20. Brusthaube nach einem der Ansprüche 1 bis 19, wobei sie einen Temperatursensor aufweist.

## Claims

1. Breast shield (4) for use with a breastpump (1) for expressing breastmilk, in which the breast shield (4) comprises a breast-shield funnel (40) for application to a mother's breast, a first coupling part (42) for connection to the breastpump (1), and a second coupling part (43) for connection to a milk collection container (44), and in which the breast shield (4) also has an electrical resistance heating element (5) for warming it, **characterized in that** the electrical resistance heating element (5) is made of an electrically conductive plastic material, and in which the breast shield (4) is produced in a two-component or multi-component injection-molding operation.

2. Breast shield as claimed in claim 1, in which the electrically conductive plastic material comprises a plastic base material and an electrically conductive additive.

3. Breast shield as claimed in claim 2, in which the additive is fibrous.

4. Breast shield as claimed in claim 3, in which the fibers have a length of 100 to 300 µm.

5. Breast shield as claimed in one of claims 2 through 4, in which the additive is one or more materials from the following group: carbon fibers, copper fibers, iron fibers.

6. Breast shield as claimed in claim 2, in which the additive is powdery.

7. Breast shield as claimed in claim 6, in which the additive is one or more materials from the following group: iron powder, copper powder, soot.

8. Breast shield as claimed in one of claims 2 through 7, in which the plastic base material is polyamide (PA), polypropylene (PP) or polyethylene (PE).

9. Breast shield as claimed in one of claims 2 through 8, in which the additive is admixed to the plastic base material in a quantity of 10% to 30% (percent by weight).

10. Breast shield as claimed in one of claims 1 through 9, in which the breast-shield funnel (40) has the resistance heating element.

11. Breast shield as claimed in claim 10, in which the resistance heating element (5) is embedded in the material of the breast-shield funnel (40).

12. Breast shield as claimed in claim 10, in which the resistance heating element (5) lies on the material of the breast-shield funnel (40).

13. Breast shield as claimed in claim 10, in which the breast-shield funnel (40) is made of polypropylene (PP), polyamide (PA) or polyethylene (PE).

14. Breast shield as claimed in one of claims 1 through 13, in which the breast shield (4) is formed in one piece, except for the resistance heating element (5).

15. Breast shield as claimed in one of claims 1 through 13, in which the breast-shield funnel (40) has a rigid area (400) and a soft area (401), and in which the resistance heating element (5) is arranged in the soft area (401).

16. Breast shield as claimed in one of claims 1 through 15, in which the breast-shield funnel (40) has an electrical plug-in connection (6).

17. Breast shield as claimed in one of claims 1 through 15, in which the first coupling part (42) has an electrical plug-in connection (22).

18. Breast shield as claimed in claim 17, in which the electrical plug-in connection (22) is arranged adjacent to a suction connection (21).

19. Breast shield as claimed in one of claims 1 through 18, in which the electrical resistance heating element (5) is arranged in a spiral or circular shape on the breast-shield funnel (40).

20. Breast shield as claimed in one of claims 1 through 19, in which it has a temperature sensor.

## Revendications

1. Téterelle (4) destinée à l'utilisation avec un tire-lait (1) pour tirer du lait maternel, la téterelle (4) présentant un entonnoir de téterelle (40) pour l'application contre un sein maternel, une première partie d'accouplement (42) pour la connexion au tire-lait (1) et une deuxième partie d'accouplement (43) pour la connexion à un récipient collecteur de lait (44), la téterelle (4) présentant en outre un élément chauffant à résistance électrique (5) pour son chauffage, **caractérisée en ce que** l'élément chauffant à résistance électrique (5) se compose d'un matériau en plastique conducteur de l'électricité et la téterelle (4) étant fabriquée en un procédé de moulage par injection à deux ou plusieurs composants.

2. Téterelle selon la revendication 1, dans laquelle le matériau en plastique conducteur de l'électricité contient un matériau de base en plastique et un additif conducteur de l'électricité.

3. Téterelle selon la revendication 2, dans laquelle l'additif est sous forme fibreuse.

4. Téterelle selon la revendication 3, dans laquelle les fibres ont une longueur de 100 à 300 µm.

5. Téterelle selon l'une quelconque des revendications 2 à 4, dans laquelle l'additif est constitué d'un ou de plusieurs matériaux dans le groupe des fibres de carbone, des fibres de cuivre, ou des fibres de fer.

6. Téterelle selon la revendication 2, dans laquelle l'additif est pulvérulent.

7. Téterelle selon la revendication 6, dans laquelle l'additif est constitué d'un ou de plusieurs matériaux dans le groupe de poudre de fer, de poudre de cuivre, ou de suie.

8. Téterelle selon l'une quelconque des revendications 2 à 7, dans laquelle le matériau de base en plastique est du polyamide (PA), du polypropylène (PP) ou du polyéthylène (PE).

9. Téterelle selon l'une quelconque des revendications 2 à 8, dans laquelle l'additif est mélangé dans une quantité de 10% à 30% (pourcentage en poids) au matériau de base en plastique.

10. Téterelle selon l'une quelconque des revendications 1 à 9, dans laquelle l'entonnoir de téterelle (40) présente l'élément chauffant à résistance.

11. Téterelle selon la revendication 10, dans laquelle l'élément chauffant à résistance (5) est encastré dans le matériau de l'entonnoir de téterelle (40).

12. Téterelle selon la revendication 10, dans laquelle l'élément chauffant à résistance (5) s'applique sur le matériau de l'entonnoir de téterelle (40).

13. Téterelle selon la revendication 10, dans laquelle l'entonnoir de téterelle (40) se compose de polypropylène (PP), de polyamide (PA) ou de polyéthylène (PE).

14. Téterelle selon l'une quelconque des revendications 1 à 13, dans laquelle la téterelle (4) est réalisée d'une seule pièce avec le logement de l'élément chauffant à résistance (5).

15. Téterelle selon l'une quelconque des revendications 1 à 13, dans laquelle l'entonnoir de téterelle (40) présente une région rigide (400) et une région souple (401) et dans laquelle l'élément chauffant à résistance (5) est disposé dans la région souple (401).

16. Téterelle selon l'une quelconque des revendications 1 à 15, dans laquelle l'entonnoir de téterelle (40) présente une connexion électrique par enfichage (6).

17. Téterelle selon l'une quelconque des revendications 1 à 15, dans laquelle la première partie d'accouplement (42) présente une connexion électrique par enfichage (22).

18. Téterelle selon la revendication 17, dans laquelle la connexion électrique par enfichage (22) est disposée à côté d'une connexion de succion (21).

19. Téterelle selon l'une quelconque des revendications 1 à 18, dans laquelle l'élément chauffant à résistance électrique (5) est disposé en spirale ou en cercle sur l'entonnoir de téterelle (40).

20. Téterelle selon l'une quelconque des revendications 1 à 19, présentant un capteur de température.
